# EUROPÄISCHE PATENTSCHRIFT

(11) **EP 4 362 874 B1**
(45) Veröffentlichungstag und Bekanntmachung des Hinweises auf die Patenterteilung: **23.09.2026**
(21) Anmeldenummer: 22734897.6
(22) Anmeldetag: 09.06.2022
(51) Int. Cl.: A61F 13/514

(54) **DRUCKPRÄGUNG**
PRESSURE-EMBOSSING
GAUFRAGE SOUS PRESSION

(30) Priorität: 29.06.2021 DE 102021116731
(43) Veröffentlichungstag der Anmeldung: 08.05.2024
(73) Patentinhaber: RKW SE, 68309 Mannheim (DE)
(72) Erfinder: MAIER, Leonhard, 83547 Babensham (DE)
(74) Vertreter: Busch, Tobias
(86) Internationale Anmeldenummer: PCT/EP2022/065639
(87) Internationale Veröffentlichungsnummer: WO 2023/274677

(56) Entgegenhaltungen:
- WO-A1-2020/142433
- US-A1- 2013 202 828
- US-A1- 2017 029 991

## Beschreibung

Die Erfindung betrifft eine polyolefinische Durchnässungsschutzlage in Hygieneprodukten mit mindestens einer Schicht, die Hohlräume aufweist.

Unter dem Begriff Hygieneprodukte im Sinne der Erfindung werden insbesondere Windeln für Babys sowie Inkontinenzprodukte für Erwachsene, als auch Damenhygieneprodukte wie beispielsweise Damenbinden zusammengefasst. Bekannte Hygieneprodukte dieser Art besitzen eine flächige, sich über das ganze Produkt erstreckende Schutzlage gegen Durchnässung. Diese Durchnässungsschutzlage dient zum Schutz der umgebenden Kleidung, um ein Austreten von Flüssigkeiten und eine Kontamination der Kleidung zu verhindern.

Bei Einwegwindeln wird die Außenseite aus einer flüssigkeitsundurchlässigen Folie gebildet, die verhindert, dass Ausscheidungen austreten. Die Folie bildet in Form eines flächigen Gebildes eine Schicht, die vom Träger abgewandt angeordnet ist und als "Backsheet" bezeichnet wird. Während in den Anfängen gasundurchlässige Kunststofffolien verwendet wurden, werden bereits seit Längerem atmungsaktive Folien eingesetzt. Diese flüssigkeitsundurchlässigen, aber gleichzeitig wasserdampfdurchlässigen Folien verbessern den Tragekomfort der Windel deutlich, da die Atmungsaktivität ein Selbsttrocknen der Windeln ermöglicht.

Bei Hygieneprodukten im Sinne der Erfindung ist ein Saugkörper auf einem polyolefinischen Vlies-Folienverbund angeordnet. Die Backsheetfolie trägt insoweit den Saugkörper, der bei modernen Windeln oft mit Superabsorbermaterial angereichert ist und dichtet ihn nach außen hin ab. Die Innenseite des Hygieneprodukts bildet üblicherweise eine Topsheetfolie aus einem flüssigkeitsdurchlässigen Material. Die Topsheetfolie dient zum Fixieren des Saugkerns nach innen hin und erhöht den Tragekomfort des Hygieneprodukts.

Heute sollte eine Backsheetfolie eine textile Anmutung aufweisen. Dies wird durch einen Faserflor auf der polyolefinischen Folie erreicht. Eine solche Kombinationsfolie ist mit einem Vliesstoff kaschiert, um eine derartige, textile Anmutung zu erhalten.

Eine weitere Anforderung an eine polyolefinische Durchnässungsschutzlage besteht in der Mindestzugfestigkeit, die für eine Verarbeitung der Folienbahn auf den enorm schnell laufenden Windel-Herstellungsmaschinen (Konvertern) erforderlich ist. Der Durchsatz der erzeugten Windeln nimmt kontinuierlich zu. Waren vor einigen Jahren noch Geschwindigkeiten von 600 Windeln pro Minute üblich, werden heute Anlagen für 1000 Windeln pro Minute und mehr eingesetzt. Mit steigender Liniengeschwindigkeit wirken höhere Kraftspitzen auf die Durchnässungsschutzlage. Somit werden an die Folien sehr hohe Anforderungen, insbesondere beim Falten der fertigen Windeln und der anschließenden Kompression gestellt, wenn die Luft aus der Windel gedrückt wird.

Die WO 00 2020 225165 A1 beschreibt eine moderne, atmungsaktive Folie, die den genannten Ansprüchen genügt. Die spezifische Ball-Drop-Impact-Fallhöhe der Folie beträgt mehr als 100 mm je Gramm Polymer pro Quadratmeter. Die Folie weist eine Wasserdampfdurchlässigkeit von mindestens 1000 g/m² in 24 Stunden auf. Die Reißdehnung der Folie in Maschinenrichtung beträgt weniger als 200 %.

Die WO 2020/142433 A1 betrifft einen Artikel, der mindestens eine erste Schicht mit einer ersten und einer zweiten Hauptfläche und eine zweite Schicht umfasst, die an die zweite Hauptfläche der ersten Schicht angrenzt. Die erste Hauptfläche der ersten Schicht ist mindestens ein Teil der sichtbaren Außenfläche des Artikels. Mindestens ein Teil der ersten Schicht ist eine durchsichtige thermoplastische Folie, die eine erste Farbe hat, die nicht weiß ist. Die zweite Schicht besteht aus einer mikroporösen Folie mit einem undurchsichtigen, mikroporösen Bereich und einem nicht porösen Bereich. Wenn der undurchsichtige, mikroporöse Bereich und der nichtporöse Bereich durch die erste Schicht hindurchgesehen werden, erzeugen sie auf der Außenfläche das Aussehen von zwei verschiedenen Farben oder zwei verschiedenen Farbtönen der gleichen Farbe.

Die US 2017/029991 A1 offenbart eine Mehrkomponentenfaser, die mindestens eine erste und eine zweite Komponente enthält. In einigen Fällen ist mindestens ein Teil der ersten Komponente undurchsichtig und mikroporös, und die zweite Komponente unterscheidet sich von der ersten Komponente. In einigen Fällen ist mindestens ein Teil der zweiten Komponente durch mindestens einen Teil der ersten Komponente hindurch sichtbar. In einigen Fällen hat das Faservlies mindestens einen ersten Bereich, in dem erste Teile der Mehrfachfasern undurchsichtig und mikroporös sind, und mindestens einen zweiten Bereich, in dem zweite Teile der Mehrfachfasern einen durchsichtigen Bereich mit geringerer Porosität bilden.

Die US 2013/202828 A1 betrifft ein Verfahren zum Drucken auf ein mikroporöses Substrat, bei dem ein Laser verwendet wird, um das Substrat zu schmelzen oder zu erweichen, so dass die Poren kollabieren und klare Bereiche auf einem weißen Hintergrund entstehen. Ein bevorzugtes Substrat ist ein Substrat auf der Basis von Polypropylen, bei dem die Mikroporen durch Ausrichten einer extrudierten Vorläuferfolie, die die beta-kristalline Form von Polypropylen enthält, erzeugt werden. Auf der nicht laserbehandelten Seite der Folie kann eine dunkle coextrudierte Schicht oder ein pigmentierter Klebstoff aufgebracht werden, so dass die behandelte Seite durch die klaren Bereiche hindurch die Farbe der hinteren Schicht zeigt. Diese Art der Bedruckung oder Lasermarkierung erfordert keine Tinten, Lösungsmittel oder andere käufliche Zusätze, so dass die Bedruckung mit sehr hohen Produktionsraten und zu geringen Kosten durchgeführt werden kann. Die geringe Porengröße der Folie ermöglicht feine Druckdetails und einen hervorragenden Druckkontrast.

Zur Erzeugung eines ansprechenden Designs werden Backsheetfolien bevorzugt mit herstellerkennzeichnenden und zielgruppengerechten Druckmotiven beschichtet. Die für die Druckfarben eingesetzten Pigmente und Stoffkombinationen, beispielsweise TiO₂, stehen zunehmend dem Anspruch von Konsumenten entgegen, Schadstoffe zu vermeiden und einen Hautkontakt mit diesen Stoffen zu verhindern.

Aufgabe der Erfindung ist es, eine polyolefinische Durchnässungsschutzlage mit einem gefälligen Design zur Verfügung zu stellen. Die Durchnässungsschutzlage soll mit einem anspruchsvollen Druckbild sowie mit einer besonderen Art des Designs überzeugen und gleichzeitig den Einsatz von Schadstoffen vermeiden. Dabei soll die Durchnässungsschutzlage besonders flüssigkeitsdicht sein, die geforderten mechanischen Eigenschaften erfüllen und einen angenehmen Tragekomfort gewährleisten. Weiterhin soll die Durchnässungsschutzlage haptisch ansprechend sein. Die Durchnässungsschutzlage soll die Qualität des Hygieneproduktes steigern und den Anforderungen bei deren Herstellung in modernen Prozessen genügen.

Diese Aufgabe wird erfindungsgemäß durch eine polyolefinische Durchnässungsschutzlage in Hygieneprodukten gelöst. Bevorzugte Varianten sind den Unteransprüchen, der Beschreibung, dem Ausführungsbeispiel und der Zeichnung zu entnehmen.

Erfindungsgemäß umfasst die Schicht der Durchnässungsschutzlage einen ersten Bereich und mindestens einen weiteren Bereich, wobei das mittlere Volumen der Hohlräume im ersten Bereich größer ist als in dem weiteren Bereich, wobei der Volumenanteil aller Hohlräume innerhalb des ersten Bereichs mehr als 10 % beträgt. Die Durchnässungsschutzlage ist eine flächig ausgebildete Folie mit besonderen Eigenschaften, die mindestens eine Schicht aufweist. In bevorzugten Varianten der Erfindung ist die Durchnässungsschutzlage zwei- oder dreischichtig ausgebildet.

Ein Hohlraum im Sinne einer Pore ist eine sehr kleine Öffnung, Höhlung oder Vertiefung. Die Größe dieser Hohlräume werden unterschieden und führen zu einer Einteilung in Mikro-, Meso- und Makroporen. Das Vorhandensein von Poren in mindestens einer Schicht führt zu Eigenschaften, die man mithilfe der Porosität der Schicht beschreiben kann.

Die Porosität ist eine dimensionslose Messgröße und stellt das Verhältnis von Hohlraumvolumen zu Gesamtvolumen eines Stoffes oder Stoffgemisches dar. Sie dient als klassifizierendes Maß für die tatsächlich vorliegenden Hohlräume.

Der erste Bereich der Schicht weist zumindest mehrere Poren auf, die eine Abmessung in der Größenordnung oder größer als die Wellenlänge des sichtbaren Lichts haben. Beispielsweise sollten zumindest einige der Poren eine Abmessung bzw. einen Durchmesser von mindestens 400 Nanometern aufweisen.

Die Größe der Hohlräume kann durch Messen des Blasenpunktes gemäß ASTM F-316-80 ermittelt werden. Die Poren können offenzellige Poren oder geschlossenzellige Poren sein. In einigen Ausführungsformen sind die Poren geschlossenzellige Poren.

Der erste Bereich der Schicht umfasst demnach Mikroporen, die dazu führen, dass der erste Bereich opak erscheint. Die Opazität ist gegensätzlich zur Transparenz. Sie ist ein Maß für die Lichtundurchlässigkeit bzw. Blickdichtigkeit in Prozent. Insbesondere liegt die Opazität einer vollkommen lichtundurchlässigen Lage bei 100 % und eine vollständig bzw. vollkommen transparente Lage weist eine Opazität von 0 % auf.

Insbesondere sind opake Bereiche von polyolefinischen Durchnässungsschutzlagen gerade nicht transparent im Wellenlängenbereich des sichtbaren Lichts. Vorteilhafterweise weist der erste Bereich der Schicht eine Opazität nach DIN 53416 von mehr als 45 %, vorzugsweise mehr als 60 %, insbesondere mehr als 75 % auf.

Der weitere Bereich der Schicht, dessen mittleres Volumen der Hohlräume kleiner als im ersten Bereich ist, weist keine Poren oder Porengrößen auf, die kleiner als die Wellenlänge des sichtbaren Lichts ausgebildet sind. Sie bewirken, dass der weitere Bereich durchsichtig erscheint.

Der Begriff durchsichtig bezieht sich entweder auf transparent, wobei der Bereich Licht durchlässt und eine klare Sicht auf dahinterliegende Schichten ermöglicht oder durchscheinend, wobei der Bereich Licht durchlässt, jedoch keine klare Sicht auf die Schichten dahinter realisiert. Der durchsichtige Bereich kann farbig oder farblos ausgebildet sein, um ein gefälliges Design zu erzeugen.

Gemäß der Erfindung weisen der erste und der weitere Bereich nahezu die gleiche Anzahl an Hohlräumen auf, wobei sich deren Volumen jedoch deutlich unterscheidet. Nahezu die gleiche Anzahl an Hohlräumen bezieht sich auf eine Differenz kleiner 10 %. In einer alternativen Ausführung der Erfindung unterscheidet sich die Anzahl der Hohlräume in den Bereichen deutlich, weil durch das Druckprägen die Hohlräume im weiteren Bereich vollständig kollabiert sind.

Vorzugsweise ist das mittlere Volumen der Hohlräume im ersten Bereich größer als in einem weiteren Bereich. Dabei kann sich das Volumen einzelner Hohlräume in den Bereichen als auch das Gesamtvolumen aller Hohlräume unterscheiden. Prinzipiell wäre es möglich, dass die Bereiche eine unterschiedliche Anzahl an Hohlräumen aufweisen. Unter Umständen können die Bereiche eine nahezu gleiche Anzahl an Hohlräumen aufweisen.

In einer äußerst vorteilhaften Ausführung der Erfindung beträgt der Volumenanteil aller Hohlräume innerhalb des ersten Bereichs mehr als 10 %, vorzugsweise mehr als 20 %, insbesondere mehr als 30 %. Der Volumenanteil des ersten Bereichs ist größer ausgebildet als der in einem weiteren Bereich, wobei der Volumenanteil aller Hohlräume innerhalb des weiteren Bereichs weniger als 70 %, vorzugsweise weniger als 20 %, insbesondere weniger als 10 % des Volumenanteils des ersten Bereichs beträgt. Auf diese Weise ist der erste Bereich der Schicht opak ausgebildet, während der weitere Bereich durchscheinend bis transparent ausgestaltet ist.

Zur Erzeugung der Hohlräume ist die Schicht der Durchnässungsschutzlage entweder β-nukleiert, mit einem anorganischen Füllstoff gefüllt oder sowohl β-nukleiert als auch gefüllt ausgebildet. Die Nukleierungsmittel und/oder die Füllstoffe werden der Polymermischung zugegeben, zu einer Folie vorzugsweise blasextrudiert und anschließend gereckt zur Ausbildung der erfindungsgemäßen Hohlraumstrukturen der Schicht.

Erfindungsgemäß werden in einer Variante der Erfindung mindestens einer Schicht der Durchnässungsschutzlage Nukleierungsmittel eingesetzt. Eine Einstellung von Sphärolitgrößen der ausgewählten Polymere mit einer Verstreckung führt zu einer Opazität, die ein ansprechendes Druckdesign sicherstellt, bei gleichzeitiger Gewährleistung besonders günstiger mechanischer Eigenschaften der Durchnässungsschutzlage.

Insbesondere bei isotaktischem Polypropylen besteht die Möglichkeit, mit geeigneten Nukleierungsmitteln gezielt die α-Kristallmodifikation oder die β-Kristallmodifikation zu nukleieren. α- und β-nukleiertes Polypropylen unterscheidet sich stark in den mechanischen und optischen Eigenschaften. Darüber hinaus gelingt es mit einigen wenigen β-Nukleierungsmitteln auch die optischen Eigenschaften so zu verbessern, dass eine opake Polypropylen-Schicht erhalten werden kann.

In einer vorteilhaften Variante der Erfindung weist mindestens eine Schicht der Durchnässungsschutzlage einen Anteil an β-Sphärolith-Keimbildner auf. Hierbei beträgt der Anteil des β-Sphärolith-Keimbildner mehr als 1 ppm, vorzugsweise mehr als 2 ppm, insbesondere mehr als 3 ppm. Dabei hat sich gezeigt, dass der Einsatz an β-Sphärolith-Keimbildner sehr sparsam ausgeführt werden kann und dabei der Anteil weniger als 1000 ppm, vorzugsweise weniger als 500 ppm, insbesondere weniger als 50 ppm beträgt.

Als β-Sphärolith-Keimbildner eignet sich vorzugsweise ein Stoff mit der Bezeichnung Cinquasia Gold, ein substituiertes Chinacridon, aufgrund seiner hervorragenden Dispergierbarkeit in Polymermischungen. Dabei reichen wenige ppm an Cinquasia Gold, vorzugsweise 10 ppm an Cinquasia Gold, die in den Extruder zur Polymermischung hinzugegeben werden, um eine äußerst fein kristalline Polypropylen-Schicht zu erzeugen. Die anschließende Verstreckung der Schicht führt dazu, dass die Schicht eine starke Opazität durch das Ausbilden einer mikroporösen Struktur aufweist. Prinzipiell sind alle bekannten β-Sphärolith-Keimbildner verwendbar, als günstig haben sich dabei Chinacridone als β-Sphärolith-Keimbildner erwiesen.

Die Durchnässungsschutzlage wird entweder nur in Maschinenrichtung (MD), oder sowohl Maschinen- als auch Querrichtung (CD) gereckt. Dies erzeugt eine mikroporöse Struktur in der Schicht, die zu der gewünschten Opazität führt.

Die Durchnässungsschutzlage kann beispielsweise mit einem Reckverhältnis von 1:2 in Maschinenrichtung verstreckt werden. Es ist auch möglich, die Folienbahn zusätzlich einer Querverstreckung (CD) zu unterziehen.

In einer weiteren, vorteilhaften Variante der Erfindung weist die Schicht einen anorganischen Füllstoff auf, beispielsweise CaCO₃, wobei der Anteil an anorganischem Füllstoff mehr als 20 Gew.-%, vorzugsweise mehr als 30 Gew.-%, insbesondere mehr als 40 Gew.-% und/oder weniger als 90 Gew.-%, vorzugsweise weniger als 80 Gew.-%, insbesondere weniger als 70 Gew.-% beträgt.

Bei einer alternativen Variante der Erfindung wird eine ausreichende Opazität ohne den Einsatz eines anorganischen Füllstoffs in der Durchnässungsschutzlage erzeugt, vorzugsweise ohne Einbindung eines Weißpigments. Erfindungsgemäß kann insbesondere auf Titandioxid verzichtet werden. Dies ist sowohl aus gesundheitlichen als auch aus ökologischen Aspekten vorteilhaft.

In einer äußerst vorteilhaften Ausführung der Erfindung weist die Durchnässungsschutzlage kein Titandioxid sowie vorzugsweise kein Weißpigment zur Erzeugung einer Opazität auf. Diese Ausführungsvariante wird den Ansprüchen des Umwelt- und des Gesundheitsschutzes in besonderem Maße gerecht. Die so erzeugte Durchnässungsschutzlage ermöglicht eine schadstofffreies Hygieneprodukt, das den Verbrauchern ein gutes Gefühl beim Tragen desselbigen ermöglicht.

Die ausgeprägte Opazität der polypropylenbasierten Durchnässungsschutzlage kann alternativ auch mit einem Aufdruck beschichtet werden. Die erfindungsgemäße Durchnässungsschutzlage zeichnet sich dadurch aus, dass sie völlig frei von anorganischen Füllstoffen und üblich eingesetzten Weißpigmenten hergestellt werden kann.

Das Druckprägen ist ein Verfahren zur Erzeugung eines ansprechenden Designs ohne das Aufbringen eines Aufdrucks bzw. einer Druckschicht auf der Außenseite der Durchnässungsschutzlage. Bei Temperaturen von 120 - 150 °C und einer Flächenpressung in den Bereichen von 5 - 25 bar werden in den weiteren Bereichen die Hohlräume verkleinert oder sogar entfernt. Das Verfahren des Druckprägens führt zum Kollabieren der Poren in den weiteren Bereichen der Schicht und kann dazu führen, dass die Anzahl und das Volumen der Hohlräume gegen null gehen. Darüber hinaus sind mikroporöse, opake und durchsichtige Porenstrukturen bei einem Band, das einen Kleber und eine Releasebeschichtung aufweist, bekannt und in der EP 3 007 666 B1 beschrieben.

Die Flächenpressung beim Druckprägen wird durch gegenüberlegende, temperierte Walzen erzeugt. Die Walzen sind hierzu auf 70 - 155 °C, vorzugsweise auf 110 - 150°C, insbesondere auf 125 - 145 °C erhitzt und erzeugen in Abhängigkeit der Foliendicke eine Flächenpressung von 5 - 500 bar, vorzugsweise von 10 - 200 bar, insbesondere von 15 - 50 bar. Das Druckprägen des weiteren Bereichs der Schicht führt dazu, dass der weitere Bereich durchscheinend bis transparent ausgestaltet ist, während der erste Bereich weiterhin opak erscheint.

Beim Druckprägen ist eine erste Walze beheizt und wirkt mit einer Gegenwalze, die die entsprechenden Negative der Motive aufweist und ebenfalls beheizt sein kann, zur Erzeugung des Druckprägemotivs zusammen. Die Durchnässungsschutzlage erfährt im Walzenspalt eine Flächenpressung, die zum Kollabieren der Hohlräume im weiteren Bereich führt. Im Nachgang kann zusätzlich ein Prägevorgang mit weiteren Walzen erfolgen.

Die Gestaltung der Gegenwalze mit entsprechenden Negativen der Druckmotive bzw. des Designs führt zu einer optisch ansprechenden Gestaltung der Durchnässungsschutzlage ohne das Aufbringen von Druckfarbe und eines Hautkontakts von eventuell schadhaften Stoffen.

Die farbliche Variation eines ansprechenden Designs der Durchnässungsschutzlage ist hierbei vielfältig ausgestaltbar. So kann beispielsweise die Schicht selbst eingefärbt sein. Das Kollabieren der Hohlräume im weiteren Bereich der Schicht lässt die Farbnuance der Einfärbung deutlich dunkler erscheinen.

Darüber hinaus kann die Schicht hinter der ersten Schicht eine Einfärbung aufweisen, wobei das Kollabieren der Hohlräume im weiteren Bereich der ersten Schicht die Einfärbung der zweiten Schicht durchscheinen lässt.

In einer alternativen Variante der Erfindung ist ein Aufdruck zwischen einer ersten Schicht und einer zweiten Schicht der Durchnässungsschutzlage aufgebracht, der durch die transparenten Bereiche der ersten Schicht durchscheint.

In einer bevorzugten Ausführungsform der Erfindung ist die Durchnässungsschutzlage dreischichtig ausgebildet, wobei die mittlere Schicht eine Einfärbung aufweist, die durch den transparenten, weiteren Bereich der ersten Schicht hindurchscheint.

Das so erzeugte Design umfasst vorzugsweise Motive und/oder Buchstaben und/oder Zahlen und/oder herstellkennzeichnende Zeichen und/oder optische Wiedererkennungssymbole. Darüber hinaus sind Muster und Strukturen ausführbar, die beispielsweise schlangenlinienförmig, zackenförmig, hexagonal, diamantenförmig, rautenförmig, parallelogrammförmig, wabenförmig, kreisförmig, punktförmig, sternförmig, leinenförmig, netzförmig, mehreckig, vorzugsweise dreieckig, viereckig, besonders bevorzugt rechteckig und quadratisch, fünfeckig, sechseckig, siebeneckig und achteckig, drahtförmig, ellipsenförmig, oval und gitterförmig gestaltet sind.

Bei einer Variante der Erfindung ist die Durchnässungsschutzlage mit Hilfe von Wärme und/oder Druck unwiderruflich markiert. Dies ist eine Möglichkeit, um Artikel vor Produktpiraterie zu schützen. Anders als eine Bedruckung, die mit einem geeigneten Lösemittel entfernt werden kann, ist es bei einer derart markierten Anordnung nicht möglich, die gezielt durch Druck und Wärme zerstörte Porenstruktur wiederherzustellen.

Für eine solche Einwirkung zur Veränderung der Porenstruktur kann Laserstrahlung eingesetzt werden. Alternativ oder ergänzend kann die Porenstruktur durch Einwirkung von Ultraschall verändert werden.

Bei einer Variante der Erfindung weist die Durchnässungsschutzlage eine Prägestruktur auf.

Die Prägestruktur beruht vorzugsweise auf einem wiederkehrenden und/oder regelmäßig angeordneten Muster. Bei der Prägestruktur kann es sich dabei um eine durchgehende Prägestruktur, wie z.B. eine durchgehende Rillenstruktur oder um mehrere vorzugsweise wiederkehrende Einzelprägestrukturen handeln.

Die Durchnässungsschutzlage weist eine Wassersäule nach EDANA WSP 80.6 von mehr als 200 mm, vorzugsweise mehr als 1000 mm, insbesondere mehr als 2000 mm auf und ist somit äußerst dicht hinsichtlich eines Flüssigkeitsdurchtritts ausgebildet.

Gleichzeitig ist die Durchnässungsschutzlage meist vorteilhaft atmungsaktiv ausgebildet und weist eine Wasserdampfdurchlässigkeit von mindestens 500 g/m² in 24 h nach ASTM D6701-01 auf. Dadurch lässt sich die Ausbildung eines Hygieneartikels mit besonders hohem Tragekomfort erzielen.

In einer alternativen Variante der Erfindung ist die Durchnässungsschutzlage nicht atmungsaktiv ausgebildet.

Das Flächengewicht der Durchnässungsschutzlage beträgt mehr als 6 g/m², vorzugsweise mehr als 10 g/m², insbesondere mehr als 15 g/m² und/oder weniger als 80 g/m², vorzugsweise weniger als 70 g/m², insbesondere weniger als 60 g/m².

Diese Flächengewichte führen zu entsprechenden Dicken der Durchnässungsschutzlage, wobei die Dicke mehr als 6 µm, vorzugsweise mehr als 10 µm, insbesondere mehr als 15 µm und/oder weniger als 85 µm, vorzugsweise weniger als 80 µm, insbesondere weniger als 75 µm beträgt.

Die erfindungsmäße Durchnässungsschutzlage kann mit einem Nonwoven verbunden sein. Weiterhin kann das Lagen-Nonwoven-Laminat mit elastischen Ohren, sogenannten Front- bzw. Back-Ears versehen werden. Diese können entweder an die Folie angeklebt oder auch thermobondiert werden.

Bei einer besonders vorteilhaften Variante der erfindungsgemäßen Durchnässungsschutzlage beträgt der Anteil an Polyolefinen mehr als 40 Gew.-%., vorzugsweise mehr als 55 Gew.-%., insbesondere mehr als 70 Gew.-%.

Bei einer bevorzugten Variante der Erfindung kommen zur Herstellung der erfindungsgemäßen Schicht der Durchnässungsschutzlage zwei unterschiedliche Polypropylen (PP)-Komponenten zum Einsatz. Dabei wird eine niedrig schmelzende PP-Komponente mit einem hoch schmelzenden Polypropylen kombiniert.

In einer äußerst vorteilhaften Variante der Erfindung weist die Schicht der Durchnässungsschutzlage mindestens 30 Gew.-% eines niedrig schmelzenden Polypropylens mit einem DSC-Schmelzpunkt zwischen 135 bis 145 °C und mindestens 8 Gew.-% eines hoch schmelzenden Polypropylens mit einem DSC-Schmelzpunkt zwischen 155 bis 165 °C auf.

Bei einer günstigen Ausführung der Erfindung umfasst die Schicht der Durchnässungsschutzlage eine Low-Density-Polyethylen (LDPE)-Komponente. Der Anteil dieser LDPE-Komponente beträgt vorzugsweise mehr als 0,5 Gew.-%, bevorzugt mehr als 1 Gew.-% und insbesondere mehr als 2 Gew.-%. Die LDPE-Komponente ist dabei vorzugsweise in einem Anteil von weniger als 12 Gew.-%, bevorzugt weniger als 8 Gew.-%, insbesondere 6 Gew.-% vorhanden.

Zur Herstellung der polyolefinischen Durchnässungsschutzlage wird eine erfindungsgemäße Zusammensetzung coextrudiert, vorzugsweise mittels Blasextrusion. Anschließend werden in einem nachgeschalteten Reckprozess mindestens in der ersten Schicht Hohlräume erzeugt. Die Erzeugung der Bereiche mit unterschiedlichem Hohlraumvolumen erfolgt durch das temperierte Druckprägen der Durchnässungsschutzlage in einem Walzenpaar. In den Bereichen, die eine temperierte Flächenpressung der Walzen erfahren, wird das mittlere Volumen der Hohlräume deutlich reduziert, wodurch die Bereiche durchscheinend bis transparent erscheinen. Die Ausgestaltung der Walzenpaarung ordnet die opaken und transparenten Bereiche zu einem Muster an, wobei die Muster in Form von Motiven und/oder von Symbolen und/oder von Zahlen und/oder von Buchstaben und/oder von Kombinationen daraus ausgebildet sind.

Die erfindungsgemäße Folie wird als Schutzlage gegen Durchnässung in Hygieneprodukten, insbesondere in Einwegwindeln, Inkontinenzprodukte und Produkten der Damenhygiene verwendet.

Weitere Vorteile und Merkmale der Erfindung ergeben sich aus der Beschreibung von Ausführungsbeispielen anhand von Zeichnungen und aus den Zeichnungen selbst. Dabei zeigt:
- Fig. 1: eine Walzenanordnung zum Druckprägen der Durchnässungsschutzlage,
- Fig. 2: eine Walzenanordnung zum Druckprägen und Prägen der Durchnässungsschutzlage.

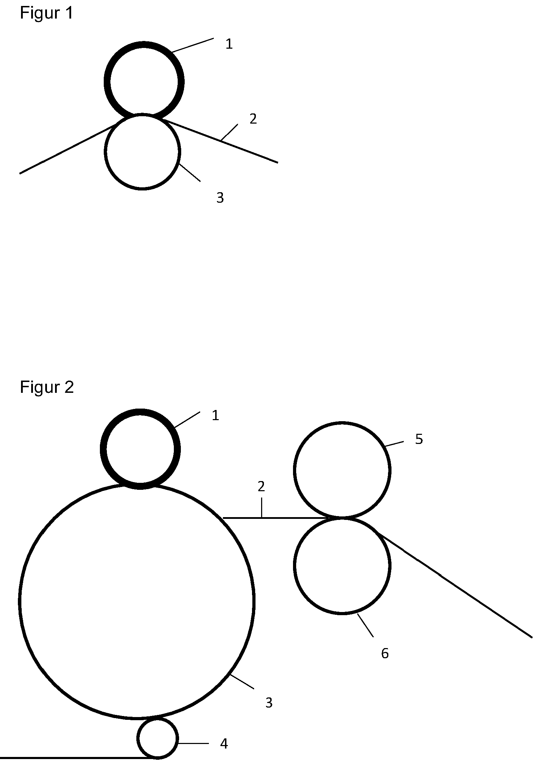

Fig. 1 zeigt eine Walzenanordnung zum Druckprägen der erfindungsgemäßen Durchnässungsschutzlage 2. Dabei wirken eine Heizwalze 3 und eine Walze mit Druckmotiven 1 durch Erhitzen und gleichzeitiges Flächenpressen auf die Durchnässungsschutzlage 2 ein, um den Volumenanteil des weiteren Bereichs der Durchnässungsschutzlage zum Kollabieren zu bringen. Die Walze mit Druckmotiven 1 ist senkrecht über der Heizwalze 3 angeordnet, wobei die Druckmotive hervorstehen und die Bereiche der Flächenpressung bilden. Dabei kann die Walze mit Druckmotiven 1 ebenfalls erhitzt werden.

Fig. 2 zeigt eine Walzenanordnung zum Druckprägen und Prägen der Durchnässungsschutzlage 2. Eine Führungswalze 4 lenkt die Durchnässungsschutzlage 2 auf die Heizwalze 3, die die Durchnässungsschutzlage 2 erhitzt. Die Walze mit Druckmotiven 1 ist senkrecht zur Heizwalze 3 angeordnet, wobei die Zentrierachsen der Walzen senkrecht zueinander ausgerichtet sind. Die hervorstehenden Druckmotive der Walze mit Druckmotiven 1 erzeugen im Walzenspalt mit der Heizwalze 3 die Flächenpressung der Durchnässungsschutzlage 2, wodurch der Volumenanteil im weiteren Bereich im Vergleich zum ersten Bereich deutlich reduziert wird. Dadurch bleibt der erste Bereich opak und der weitere Bereich bildet sich transparent bis durchscheinend aus. Die Durchnässungsschutzlage 2 erfährt zusätzlich eine Prägung, die im Walzenspalt einer Prägewalze 5 mit einer senkrecht gegenüberstehenden Gummiwalze 6 erzeugt wird.

Im Folgenden wird die Erfindung anhand eines Beispiels erläutert, ohne die Erfindung darauf zu beschränken.

Bei diesem Beispiel kommen folgende Komponenten zum Einsatz:
- 48 % Calciumcarbonat, Imerys Filmlink 400
- 32 % PP Random Copolymer, Braskem DR 155.01
- 9,6 % Calciumcarbonat, Omyafilm VL-OM PL 753
- 6,4 % PP Block Copolymer, Inspire 7056.05
- 4,0 % LDPE, DOW 310 E,
- 1 ppm blaues Pigment.

Bei dem eingesetzten Füllstoff handelt es sich um einen anorganischen Füllstoff in Form von Calciumcarbonat, vorzugsweise mit einer Partikelgröße von 0,8 bis 2 µm.

Bei dem Braskem DR 155.01 handelt es sich um ein PP Random Copolymer. Dieses PP weist einen Schmelzflussindex von 1,7 g/10min (bei 230°C /2,16kg) gemäß ASTM D1238 und einen DSC-Schmelzpunkt von 142°C auf.

Bei dem Inspire 7056.05 handelt es sich um die zweite PP-Komponente. Der Schmelzflussindex beträgt 3,0 g/10min (bei 230°C, 2,16 kg) gemäß ISO 1133.

Das LDPE DOW 310 E weist eine Dichte von 0,924 g/cm³ auf. Der Schmelzflussindex beträgt 0,7 g/10min (bei 190°C, 2,16 kg) gemäß ISO 1133.

Zur Herstellung der erfindungsgemäßen Folie werden die Polymerbestandteile mit den mineralischen Füllstoffen in einem Extruder, zum Beispiel einem Compounding-Extruder auf eine Temperatur deutlich über der Schmelztemperatur der Polymerbestandteile erhitzt (zum Beispiel über 200°C) und miteinander verschmolzen.

Dann schließt sich erfindungsgemäß eine Blasextrusion an. Bei dem Blasextrusionsverfahren wird ein Blasschlauch gebildet. Die gebildete dreischichtige Schlauchfolie kann flach aufeinander gelegt und an den Enden aufgeschlitzt werden, sodass zwei Folienbahnen entstehen.

Bei dem Blasfolienprozess kommt ein Aufblasverhältnis von 1 : 2,5 zur Anwendung.

Das Primärflächengewicht der Folie beträgt 40 g/m².

Im anschließenden monoaxialen Reckprozess wird die Folie um insgesamt 100 % in Maschinenrichtung verstreckt.

Es ergibt sich ein Flächengewicht der Folie von 22 g/m².

Die blau eingefärbte Durchnässungsschutzlage wird bei 135 °C und mit einem Flächendruck von 20 bar druckgeprägt. Dadurch erscheinen der opaken Bereich der Folie hellblau, während der druckgeprägten Bereich dunkelblau erscheint und sich ein flächiges Druckmotiv der Durchnässungsschutzlage ausbildet. In diesem Ausführungsbeispiel sind die Buchstaben RKW dunkelblau hervorgehoben.

Die Durchnässungsschutzlage weist eine Wasserdampfdurchlässigkeit nach EDANA WSP 80.6 von 1000 g/m² in 24 Stunden auf.

## Patentansprüche

1. Polyolefinische Durchnässungsschutzlage in Hygieneprodukten mit mindestens einer Schicht, die Hohlräume aufweist,
**dadurch gekennzeichnet,**
**dass** die Schicht einen ersten Bereich und mindestens einen weiteren Bereich umfasst, wobei der Volumenanteil der Hohlräume im ersten Bereich größer ist als in dem weiteren Bereich, wobei der Volumenanteil aller Hohlräume innerhalb des ersten Bereichs mehr als 10 % beträgt.

2. Durchnässungsschutzlage nach Anspruch 1, **dadurch gekennzeichnet, dass** der Volumenanteil aller Hohlräume innerhalb des ersten Bereichs mehr als 20 %, insbesondere mehr als 30 % beträgt.

3. Durchnässungsschutzlage nach Anspruch 1 oder 2, **dadurch gekennzeichnet, dass** der Volumenanteil aller Hohlräume innerhalb des weiteren Bereichs weniger als 70 %, vorzugsweise weniger als 20 %, insbesondere weniger als 10 % des Volumenanteils des ersten Bereichs beträgt.

4. Durchnässungsschutzlage nach einem der Ansprüche 1 bis 3, **dadurch gekennzeichnet, dass** die Wassersäule nach EDANA WSP 80.6 mehr als 200 mm, vorzugsweise mehr als 1000 mm, insbesondere mehr als 2000 mm beträgt.

5. Durchnässungsschutzlage nach einem der Ansprüche 1 bis 4, **dadurch gekennzeichnet, dass** die Schicht als β-nukleierte Schicht ausgebildet ist.

6. Durchnässungsschutzlage nach einem der Ansprüche 1 bis 5, **dadurch gekennzeichnet, dass** die Schicht einen Füllstoff, insbesondere einen anorganischen Füllstoff aufweist, wobei der Anteil an anorganischen Füllstoff mehr als 20 Gew.-%, vorzugsweise mehr als 30 Gew.-%, insbesondere mehr als 40 Gew.-% und/oder weniger als 90 Gew.-%, vorzugsweise weniger als 80 Gew.-%, insbesondere weniger als 70 Gew.-% beträgt.

7. Durchnässungsschutzlage nach einem der Ansprüche 1 bis 5, **dadurch gekennzeichnet, dass** die Schicht keinen anorganischen Füllstoff aufweist.

8. Durchnässungsschutzlage nach einem der Ansprüche 1 bis 7, **dadurch gekennzeichnet, dass** die Schicht als verstreckte Schicht ausgebildet ist.

9. Durchnässungsschutzlage nach einem der Ansprüche 1 bis 8, **dadurch gekennzeichnet, dass** die Durchnässungsschutzlage eine Wasserdampfdurchlässigkeit von mindestens 500 g/m² in 24 h nach ASTM D6701-01 aufweist.

10. Durchnässungsschutzlage nach einem der Ansprüche 1 bis 9, **dadurch gekennzeichnet, dass** die Schicht und/oder eine weitere Schicht einen Anteil an Farbpigmenten aufweist.

11. Durchnässungsschutzlage nach einem der Ansprüche 1 bis 10, **dadurch gekennzeichnet, dass** hinter der Schicht ein Aufdruck angeordnet ist.

12. Durchnässungsschutzlage nach einem der Ansprüche 1 bis 11, **dadurch gekennzeichnet, dass** das Flächengewicht der Durchnässungsschutzlage mehr als 6 g/m², vorzugsweise mehr als 10 g/m², insbesondere mehr als 15 g/m² und/oder weniger als 80 g/m², vorzugsweise weniger als 70 g/m², insbesondere weniger als 60 g/m² beträgt.

13. Durchnässungsschutzlage nach einem der Ansprüche 1 bis 12, **dadurch gekennzeichnet, dass** die Schicht
- mindestens 30 Gew.-% eines niedrig schmelzenden Polypropylens mit einem DSC-Schmelzpunkt zwischen 135 bis 145 °C aufweist und
- mindestens 8 Gew.-% eines hoch schmelzenden Polypropylens mit einem DSC-Schmelzpunkt zwischen 155 bis 165 °C aufweist.

14. Durchnässungsschutzlage nach einem der Ansprüche 1 bis 13, **dadurch gekennzeichnet, dass** die Schicht mindestens 3 Gew.-% eines LDPE aufweist.

15. Durchnässungsschutzlage nach einem der Ansprüche 1 bis 14, **dadurch gekennzeichnet, dass** die Bereiche zu einem Muster angeordnet sind, wobei die Muster in Form von Motiven und/oder von Symbolen und/oder von Zahlen und/oder von Buchstaben und/oder von Kombinationen daraus ausgebildet sind.

16. Durchnässungsschutzlage nach einem der Ansprüche 1 bis 15, **dadurch gekennzeichnet, dass** die Bereiche eine Markierung mittels Einwirkung von Laserstrahlung und/oder Ultraschall aufweisen.

## Claims

1. A polyolefin-based seepage protection layer for hygiene products comprising at least one layer that has cavities,
**characterized in**
**that** the layer includes a first area and at least one further area, wherein the volume fraction of the cavities in the first area is more than in the additional area, and wherein the volume fraction of all cavities within the first area is more than 10 %.

2. A seepage protection layer according to claim 1, **characterized in that** the volume fraction of all cavities within the first area is more than 20 %, and in particular more than 30%.

3. A seepage protection layer according to claim 1 or 2, **characterized in that** the volume fraction of all cavities within the additional area is less than 70 %, preferably less than 20 %, and in particular less than 10 % of the volume fraction of the first area.

4. A seepage protection layer according to any one of claims 1 to 3, **characterized in that** the water column according to EDANA WSP 80.6 is more than 200 mm, preferably more than 1000 mm, and in particular more than 2000 mm.

5. A seepage protection layer according to any one of claims 1 to 4, **characterized in that** the layer is formed as a β-nucleated layer.

6. A seepage protection layer according to any one of claims 1 to 5, **characterized in that** the layer comprises a filler, in particular an inorganic filler, wherein the proportion of inorganic filler is more than 20 % by weight, preferably more than 30 % by weight, in particular more than 40 % by weight, and/or less than 90 % by weight, preferably less than 80 % by weight, and in particular less than 70 % by weight.

7. A seepage protection layer according to any one of claims 1 to 5, **characterized in that** the layer does not include an inorganic filler.

8. A seepage protection layer according to any one of claims 1 to 7, **characterized in that** the layer comprises a stretched layer.

9. A seepage protection layer according to any one of claims 1 to 8, **characterized in that** the seepage protection layer has a water vapor transmission rate of at least 500 g/m² in 24 h according to ASTM D6701-01.

10. A seepage protection layer according to any one of claims 1 to 9, **characterized in that** the layer and/or another layer has a proportion of coloured pigments.

11. A seepage protection layer according to any one of claims 1 to 10, **characterized in that** a print is arranged behind the layer.

12. A seepage protection layer according to any one of claims 1 to 11, **characterized in that** the basis weight of the seepage protection layer is more than 6 g/m², preferably more than 10 g/m², and in particular more than 15 g/m² and/or less than 80 g/m², preferably less than 70 g/m², and in particular less than 60 g/m².

13. A seepage protection layer according to any one of claims 1 to 12, **characterized in that** the layer
- has at least 30 % by weight of a low-melting polypropylene with a DSC melting point between 135 and 145 °C and
- has at least 8 % by weight of a high-melting polypropylene with a DSC melting point between 155 and 165 °C.

14. A seepage protection layer according to any one of claims 1 to 13, **characterized in that** the layer comprises at least 3 % by weight of an LDPE.

15. A seepage protection layer according to any one of claims 1 to 14, **characterized in that** the areas are arranged in a pattern, wherein the patterns are formed in the form of motifs and/or symbols and/or numbers and/or letters and/or combinations thereof.

16. A seepage protection layer according to any one of claims 1 to 15, **characterized in that** the areas have a marking produced by the application of laser radiation and/or ultrasound.

## Revendications

1. Couche polyoléfinique de protection contre la fuite dans des produits d'hygiène, comportant au moins une couche présentant des cavités, **caractérisée en ce**
**que** la couche comprend une première zone et au moins une autre zone, la proportion volumique des cavités dans la première zone étant supérieure à celle de l'autre zone, la proportion volumique de l'ensemble des cavités à l'intérieur de la première zone étant supérieure à 10 %.

2. Couche de protection contre la fuite selon la revendication 1, **caractérisée en ce que** la proportion volumique de l'ensemble des cavités à l'intérieur de la première zone est supérieure à 20 %, en particulier supérieure à 30 %.

3. Couche de protection contre la fuite selon la revendication 1 ou 2, **caractérisée en ce que** la proportion volumique de toutes les cavités situées dans la zone supplémentaire est inférieure à 70 %, de préférence inférieure à 20 %, en particulier inférieure à 10 % de la proportion volumique de la première zone.

4. Couche de protection contre la fuite selon l'une des revendications 1 à 3, **caractérisée en ce que** la colonne d'eau selon la norme EDANA WSP 80.6 est supérieure à 200 mm, de préférence supérieure à 1 000 mm, en particulier supérieure à 2 000 mm.

5. Couche de protection contre la fuite selon l'une des revendications 1 à 4, **caractérisée en ce que** la couche est réalisée sous forme de couche à nucléation β.

6. Couche de protection contre la fuite selon l'une des revendications 1 à 5, **caractérisée en ce que** la couche comprend une charge, en particulier une charge inorganique, la proportion de charge inorganique étant supérieure à 20 % en poids, de préférence supérieure à 30 % en poids, en particulier supérieure à 40 % en poids et/ou inférieure à 90 % en poids, de préférence inférieure à 80 % en poids, et en particulier inférieure à 70 % en poids.

7. Couche de protection contre la fuite selon l'une des revendications 1 à 5, **caractérisée en ce que** la couche ne contient pas de charge inorganique.

8. Couche de protection contre la fuite selon l'une des revendications 1 à 7, **caractérisée en ce que** la couche est réalisée sous forme de couche étirée.

9. Couche de protection contre la fuite selon l'une des revendications 1 à 8, **caractérisée en ce que** la couche de protection contre la fuite présente un taux de transmission de la vapeur d'eau d'au moins 500 g/m² en 24 h selon la norme ASTM D6701-01.

10. Couche de protection contre la fuite selon l'une des revendications 1 à 9, **caractérisée en ce que** la couche et/ou une autre couche contient une proportion de pigments colorés.

11. Couche de protection contre la fuite selon l'une des revendications 1 à 10, **caractérisée en ce qu'**une impression est disposée derrière la couche.

12. Couche de protection contre la fuite selon l'une des revendications 1 à 11, **caractérisée en ce que** le grammage de la couche de protection contre l'humidité est supérieur à 6 g/m², de préférence supérieur à 10 g/m², en particulier supérieur à 15 g/m² et/ou est inférieure à 80 g/m², de préférence inférieure à 70 g/m², en particulier inférieure à 60 g/m².

13. Couche de protection contre la fuite selon l'une des revendications 1 à 12, **caractérisée en ce que** la couche
- comporte au moins 30 % en poids d'un polypropylène à bas point de fusion présentant un point de fusion DSC compris entre 135 et 145 °C et
- comporte au moins 8 % en poids d'un polypropylène à haut point de fusion présentant un point de fusion DSC compris entre 155 et 165 °C.

14. Couche de protection contre la fuite selon l'une des revendications 1 à 13, **caractérisée en ce que** la couche contient au moins 3 % en poids d'un LDPE.

15. Couche de protection contre la fuite selon l'une des revendications 1 à 14, **caractérisée en ce que** les zones sont disposées selon un motif, les motifs se présentant sous la forme de motifs décoratifs et/ou de symboles et/ou de chiffres et/ou de lettres et/ou de combinaisons de ceux-ci.

16. Couche de protection contre la fuite selon l'une des revendications 1 à 15, **caractérisée en ce que** les zones comportent un marquage réalisé par l'action d'un rayonnement laser et/ou d'ultrasons.
